# EUROPEAN PATENT APPLICATION

(11) **EP 1 952 825 A1**
(43) Date of publication of application: **06.08.2008**
(21) Application number: 06805000.4
(22) Date of filing: 20.10.2006
(51) Int. Cl.: A61K 47/48, A61K 33/26, A61K 9/14, A61K 39/395, A61P 35/00, A61K 9/08

(54) **NANOMETER TARGETED DRUG FOR MAGNETO-THERMOTHERAPY OF MALIGNANT TUMORS**

(30) Priority: 25.10.2005 CN 200510095000
(71) Applicant: Zhu, Hong, Nanjing City, Jiangsu 210037 (CN)
(72) Inventor: Zhu, Hong, Nanjing City, Jiangsu 210037 (CN)
(74) Representative: Spencer, Matthew Peter
(86) International application number: PCT/CN2006/002790
(87) International publication number: WO 2007/048326

(57) **Abstract**

The present invention relates to a targeted magnetic nanoparticle drug and the preparation method thereof. The targeted magnetic nanoparticle drug comprises an effector molecule and a guidance molecule in a weight ratio of 1 : 0.0001-0.20. The aforesaid effector molecule is a magnetic particle, with a particle size of not more than 1000 nm and a specific adsorption rate (SAR) of 10-7000 W/gFe. The aforesaid guidance molecule comprises an antibody, a ligand or a magnetic particle. The particle size of the aforesaid targeted drug is 2-1000 nm. The targeted magnetic nanoparticle drug is prepared by coupling a magnetic particle and a guidance molecule in a weight ratio of 1 : 0.0001-0.20 in water, organic or inorganic substance or the mixed solution thereof. The resultant targeted magnetic nanoparticle drug can realize targeted magnetic hyperthermia treatment and targeted magnetic thermoablation treatment and prevention on the tumors, and effectively kill the cancer cells, and cure the malignant tumors.

## Description

### FIELD OF THE INVENTION

The present invention relates to a targeted nanoparticle drug for magnetic hyperthermia treatment on malignant tumors, the preparation method and uses thereof. In particular, it relates to a targeted magnetic nanoparticle - antibody/ligand drug and a targeted magnetic nanoparticle magnetic drug with a high specific absorption rate (SAR), both for magnetic hyperthermia treatment on malignant tumors.

### BACKGROUND OF THE INVENTION

There are about 10 millions of new patients suffering malignant tumors throughout the world in 2000, with 6.2 million deads and 22 million prevalent cases. Up to 15 millions of new cases are expected for malignant tumors in 2020, with 10 million deads and 30 million prevalent cases. Malignant tumors are becoming the first killer of mankind in the new era and would cause great losses to the global economy. Therefore it is of great significance to conduct researches on the new drugs and new treatments for malignant tumors.

Traditional treatment methods for malignant tumors are primarily surgery, radiotherapy and chemotherapy. Usually only 10%-20% patients of malignant tumors have the opportunity for surgery, which ususally brings more suffering to the patients. Also it easily causes recurrence and metastasis after the surgery. Since radiotherapy and chemotherapy have no clear targeting, they will destroy normal cells while killing the tumor cells, with greater toxicity. In recent years, due to the emergence of targeted drugs of high efficiency, low toxicity, less side effects etc., the targeted drugs for effector molecules, such as an antibody and ligand coupled radionuclide, toxin, chemotherapy drugs or precursor drugs, have become new means to widely research and application of new anti-tumor drugs and treatment of malignant tumors. Research and application of targeted drugs such as an antibody and ligand have provided new opportunities to and effectiveness on the treatment and prevention of malignant tumors. However, the aforesaid effector molecules, such as radioactive nuclides, toxin, chemical drugs, or precursor drugs coupled with antibodies or ligands, still have strong toxic side effects and there are obvious limitations/defects in clinical applications. Therefore, new types of effector molecules having no toxic side effects are needed for targeted drugs for the treatment of malignant tumors.

Four types of physical and biological effects are applied in magnetic hyperthermia for malignant tumors, namely (1) magnetic thermoseed (i.e. magnetic particles or materials, such as a magnetic nanoparticle) can induct an alternating magnetic field *in vitro* to produce the magnetic heat effect; (2) the blood flow of the malignant tumors is only 10%-15% of that of normal tissues. After the tumors are heated, the blood flow of the normal tissues surrounding the tumor is accelerated, heat dissipates quickly and temperature rises slightly, while for the tumor tissues, heat dissipation is difficult, and the temperature can be 5-10 degree C higher than that of the surrounding normal tissues; (3) normal cells can die at 48 degee C or higher, and magliant cells will die at 42 degree C or higher due to their poor heat resistance; (4) alternating magnetic field at low frequency has good penetrability, without attenuation or injuries to the normal tissues, and can safely and effectively reach the inside of the body and the inside of large tumors. It also allows the magnetic thermoseeds introduced in the tumor focus to generate heat effect under the magnetic loss by the effect of the alternating magnetic field of the magnetic hyperthermia instrument to heat the magliant tumor to 42-48 degree C, basically without raising the normal tissue's temperature. As result, this is a new approach to precise positioning and selectively killing of malignant tumors without toxic side effects. In recent years, a large number of animal tests and clinical tests show that application of magnetic hyperthermia treatment on malignant tumors can not only effectively kill the tumors to make the tumor regress completely, but also pose no toxic side effects. Magnetic hyperthermia treatment is a new kind of method for treating tumor with broad application prospects, and its emergence will bring hope to safe cure of malignant tumors. A large number of researches show that the magnetic thermoseed in the magnetic hyperthermia is a physical effector molecule of targeted drug with non-toxic side effects. However, so far the magnetic thermoseeds in the magnetic hyperthermia still need to be introduced into the tumor focus by implantation and local injection, etc. There are no reports demonstrating the coupling of antibodies or ligands and magnetic thermoseeds (i.e. magnetic nanoparticle) to make an active targeted drug as well as the utilization of magnetic performace of magnetic thermoseeds to make magnetic targeted drug for magnetic hyperthermia treatment and the utilization of their active targeting function and magnetic targeting function to make drug targetedly introduced into tumor focus, for treating the malignant tumors by magnetic hyperthermia treatment.

### SUMMARY OF THE INVENTION

The object of the present invention is to overcome the disadvanges of prior art and to provide a targeted nanoparticle drug, that is, a kind of magnetic nanoparticle - antibody targeted drug, magnetic nanoparticle - ligand targeted drug, or magnetic nanoparticle magnetic targeted drug, for magnetic hyperthermia on malignant tumors with high heat-generation ability. There is also an antibody-mediated and receptor-mediated magnetic nanoparticle targeted drug, and magnetism-directed magnetic nanoparticle targeted drug with a high specific heat-generation power or a high specific absorption rate (SAR) for magnetic hyperthermia treatment on malignant tumors.

In the present invention, magnetic nanoparticle targeted drugs (including magnetic nanoparticle - antibody targeted drug, and magnetic nanoparticle - ligand targeted drug) refer to the active targeted nanoparticle drugs in which guidance molecules (such as antibodies or ligands) are coupled to magnetic nanoparticles (i.e. magnetic thermoseeds). Magnetic nanoparticle targeted drugs also refer to the magnetic targeted nanoparticle drugs for magnetic hyperthermia that made from magnetic nanoparticles through surface modification or by coating, and the SAR of the magnetic nanoparticles applied is 10-7000 W/gFe, particularly 500-7000 W/gFe.

### The present invention is achieved through the following technical solutions:

The present invention provides a targeted nanoparticle drug for magnetic hyperthermia treatment on malignant tumors, comprising an effector molecule and a guidance molecule in a weight ratio of 1 : 0.0001-0.20. The aforesaid effector molecule is a magnetic nanoparticle, with a particle size of not more than 1000 nm and SAR (specific absorption rate) of 10-7000 W/gFe. The aforesaid guidance molecule includes an antibody and a ligand as well as a magnetic particle with targeted magnetism function. The particle size of the aforesaid targeted drug is 2-1000 nm.

The aforesaid targeted nanoparticle drug for magnetic hyperthermia treatment on malignant tumors comprises a conjugate made by mixing and coupling the aforesaid magenetic nanoparticle with an antibody or a ligand in a solution, wherein the aforesaid magenetic nanoparticle is the magetic particle through surface modification or coating, or the untreated bare magnetic nanoparticle. In the conjugates, the weight ratio of magnetic nanoparticles to antibodies or ligands is 1:0.0001-0.20, and at least one or more antibodies or ligands are coupled on the surface of magnetic nanoparticles of the conjugates. The particle size of the conjugates is 2-1000 nm, with biological activity of the coupled antibody or ligand retained, possessing antibody-mediated or receptor-mediated active targeted function, magnetic targeted function, and nano-particle passive targeted function.

The magnetic nanoparticle magnetic targeted drug for magnetic hyperthermia treament on malignant tumors in the present invention substantially comprises magnetic nanoparticles made from surface modification, or coating of magnetic nanoparticles, or from the untreated bare magnetic nanoparticles. In the magnetic nanoparticles through surface modification or coating, the weight ratio of the magnetic nanoparticles to surface-modifying substances is 1 :0.0001-0.20. The weight ratio of the magnetic nanoparticles to surface-coating substances is 1 : 0.01-5.0. The particle size of modified or coated magnetic nanoparticles is 2-1000 nm, possessing magnetic targeted function and nano-particle passive targeted function.

Under the present invention, the particle size of the targeted drugs is preferably 2-400 nm. When the particle size is less than 100 nm, the targeted drug has a good long-circulating stability *in vivo* in blood, and can effectively makes use of the EPR effects (enhancing permeability and retention effects) of tumors. Generally the suitable paricle size of targeted drug is less than 100 nm.

To generate a targeted nanoparticle drug, the aforesaid conjugates or magnetic nanoparticles are mixed with a solution, which can include composite solvents and/or water (preferably deionized water). The aforesaid targeted nanoparticle drugs are primarily in injectable form since the drugs in an injection can maintain good dispersibility and stability, possess good penetrability, and have good specificity to and affinity with the antigens or receptors of malignant tumor cells. They have good targeting roles *in vitro* and *in vivo* application thereof, without cross-reaction with normal cells.

The aforesaid targeted nanoparticle drugs of the present invention are administrated through intravenous injection, intraperitoneal or direct injection (including: minimal invasion, intervention, injection, etc.), which can be directed to the malignant tumor focus position of higher specificity and affinity at high concentration. They also can be directed to the malignant tumor focus position of built-in or built-out magnets at high concentration, showing active targeting function and magnetic targeting function. Under alternating magnetic field of magnetic hyperthermia therapeutic apparatus, due to the heat effect of magnetic nanoparticle in an alternating magnetic field, the tumor focus positon which the magnetic nanoparticles in the drug directed to at high concentration is heated to 42-48 degree C, even above 48 degree C, which applys targeting thermotherapy and targeting magnetic hyperthermia treatment and prevention to the tumors, and kills the cancer cells and cures the malignant cancers effectively. For the convenience of storage, the targeted nanoparticle drugs can be made into solid or powder dosage forms.

When the particle size of the targeted drug is less than 100 nm, it has long circulating stability *in vivo* in blood and can effectively utilize the EPR effects of tumors. Therefore, the particle size of the magnetic nanoparticle in the present invention is preferably 1-400 nm, and more preferably less than 100 nm. The magnetic nanoparticles include ferromagnetic nanoparticles and ferrimagnetic nanoparticles (such as Fe₂O₃, Fe₃O₄, Fe, etc) and superparamagnetic nanoparticles (such as Fe₂O₃, Fe₃O₄ and Fe, etc, with particle size of less than the critical size).

The aforesaid magnetic nanoparticles are preferably selected from those through surface modification by surfactant/surface modifier, coating or wrapping by organic substances/inorganic substances, for one or more times so as to allow the magnetic nanoparticles to have a functionalized surface, such as an anionic, cationic, non-ionic, amino, mercapto, hydroxyl, carboxyl surface, or surface-property-philic polymeric surface as well as biomacromolecule surface and inorganic substance surface. Those are the functionalized surfaces which can be coupled with antibodies and ligands via direct interaction, small molecules, and macromolecules or via electrostatic force, hydrophobic interaction, covalent bond, van der Waals force, short-range repulsion force or adsorption action, etc. Those surfactants and surface modifiers include sodium laurylsulfate, sodium dodecyl benzene sulfonate, sodium oleate, decanoic acid, polyethylene glycol (PEG), glucan, poly-glutamic acid, carboxymethyl glucosan or amido glucosan, dextran, ferric ammonium (DF), ethylenediamine, diethylenetriamine pentaacetic acid (DTPA), hydrazinonicotinamide (HYNIC), nucleotides and polypeptides. And the coupling agents include 3-aminopropyltrimethoxysilane, aminopropyl trimethylsilane, γ-methyl acryloyloxypropyl trimethoxy silane, or other magnetic nanoparticle surface modifiers. The weight ratio of magnetic nanoparticles to surfactants or surface modifiers is 1 : 0.0001-0.20. The aforesaid organic substances include liposomes, polylactic acid, polycaprolactone, chitosan or proteins, and the inorganic substances are silicon dioxide, alumina or gold. The weight ratio of the magnetic nanoparticles to the organic/inorganic substances is 1 : 0.01-5.0.

In the present invention, the aforesaid antibody refers to an antibody against a tumor-specific antigen and a tumor-associated antigen, an idiotypic determinant, a receptor of some cytokines, a hormone or a product of some tumor gene (as a related target molecule), and includes a heterologous polyclonal antibody, a mouse monoclonal antibody (McAb) and/or a human monoclonal antibody (McAb) created from combination of immunology and cell engineering, such as a single-chain antibody (ScFv), a multivalent mini antibody, a single-domain antibody, a humanized antibody and a human antibody. Among them, antibody Fab fragments and genetically engineered antibodies with small molecular weight, high specificity and affinity are superior to those conventional monoclonal antibodies in terms of immunogenicity, penetration and the resistance to various hydrolases. They can penetrate and reach to the tumor positions much easierly than the conventional monoclonal antibodies. Those antibodies are selected from those against CEA (carcinoembryonic antigen), AFP (alpha-fetoprotein), CA19-9 (carbohydrate antigen 19-9), CA125 (cancer antigen 125), PSA (prostate soecific antigen), β-hCG (human chorionic gonadotropin), Trigen, TheraCIMh-R3, Smart ID10, Vitaxin, 4B5, SS1 (dsFv) PE38, ABX-EGF, BrevaRex, CA15-3, PAP, CA50, SCC, and β₂M (β₂-microglobulin), and Rituxan, anti-VEGF, epratuzmab, anti-NSE (neuro-specific enolase), anti-HER2, anti-TPA (tissue polypeptide antigen) antibodies and anti-human hepatoma McAb HAb18 (humanized antibody 18), or any combination thereof, or other antibodies.

The aforesaid ligands include folate ligand, transferrin proteins, carbohydrate ligand, polypeptides (such as antibody fragment), nucleic acids, hormones, and other endogenous molecules, wherein the folate ligand includes folic acid, folinic acid, dihydrofolic acid, tetrahydrofolic acid, tetrahydropterin, pteroylpolyglutamic acid, 2-deamino-hydroxy folate, 1-deamino-hydroxy folate, 1-denitrogen folate, 3-denitrogen folate, 8-denitrogen folate, 5-methyltetrahydro folate (5-CH₃N₄ folate)), 5-formyl tetrahydro folate (5-CHOH₄ folate), antifolic acid agent methotrexate (MTX), 5,10-methylene tetrahydro folate (5,10-CHH folate, DDATHF) or any combination thereof, or other ligands.

The aforesaid coupling of antibodies and magnetic nanoparticles refers to direct cross-linking method, i.e. glutaraldehyde, cis-aconitic anhydride, active ester, N-succinimidyl amide-3-(2-pyridyl dithio) propionate (SPDP), succinic anhydride, maleic anhydride, glutaric anhydride, hydrazine, oligopeptide, and hydrazone derivatives and peptide-linking methods that small molecules connect with the antibodies for the drugs, and the indirect binding cross-linking methods by the macromolecule carriers such as glucan, poly-glutamate, carboxymethyl glucosan, amino glucosan and poly-polypeptide carriers, etc.

The aforesaid coupling between ligands and magnetic nanoparticles refers to adsorption coupling via small molecules, macromolecules or direct binding, and covalent coupling such as avidin-biotin coupling, ligand-nanoparticle coupling via amide bond, and ligand-nanoparticle coupling via thioether-bond, as well as the ligand - nanoparticle coupling via Schiff-bond.

The aforesaid folate coupling usually requires ligands such as ferric ammonium (DF), diethylenetriamine pentaacetic acid (DTPA), and hydrazinonicotinamide (HYNIC), in which amino or carboxyl groups are present. Coupling is the reaction between the amino group of the ligands and the carboxyl group of folic acid. The ligands serve as bifunctional chelating agents to connect folic acid with others. Folic acid molecules need ethylenediamine or hydrazine arms if there is no amino group in the ligands.

The aforesaid mixing and coupling in a solution refers to the coupling in the water, inorganic or organic substances (such as ethanol, acetic acid, toluene, phosphate, or dimethylene sulfone) or the mixed solutions thereof. The solvents include organic substances or inorganic substances or the mixtures thereof, and the organic substances can be ethanol, acetic acid, toluene or dimethylene sulfone.

The said targeted drugs in the injection are in nanoparticle states so as to allow the targeted drugs to have good penetrability, and good specificity to and affinity with the associated tumor cells. The targeted drugs via injection will target actively and directly to the malignant tumor focus at a high concentration, which is 1-1000 times as that in the blood of normal tissues. The SAR of magnetic nanoparticles under the alternating fields of magnetic hyperthermia therapeutic apparatus is 10-7000 W/gFe among which 501-7000 W/gFe is preferred.

The aforesaid magnetic nanoparticles targeted drug injections include the injections made from targeted drugs after coupling and the injections prepared by dry solid powder of targeted drugs prior to use immediately.

The aforesaid targeted drug injections in composite solvents or water (preferably deionized water) are the formulations in which the targeted drugs dispersed in composite solvents of inorganic, organic, biological and non-biological substances, such as normal saline, phosphate buffered saline (PBS), polyethylene glycol or nucleotides, or the formulations in the water or deionized water, in which the weight ratio of targeted drugs to composite solvents or water is 1 : 0.5-100, and the weight ratio of the solute in the solvent to water is not more than 1:5.

In the present invention, magnetic nanoparticles are put forwarded as a new concept of physical effector molecules of an antibody targeted drug without toxic side effects. As a new strategy for targeted magnetic hyperthermia treatment on malignant tumors, targeted magnetic nanoparticles - antibody or ligand drugs are prepared by coupling magnetic nanoparticles as effector molecules and antibodies or ligands as the targeted guidance molecules. Those targeted drugs for magnetic hyperthermia treatment on malignant tumors by coupling magnetic nanoparticles and antibodies can target actively to the tumor focus via intravenous, arterial or intraperitoneal injection at high concentration. Under the alternating magnetic field of *in vitro* magnetic hyperthermia therapeutic apparatus, the magnetic nanoparticles concentrated at the focus will produce magnetic loss heat effect, heating the tumors to 42-48 degree C and above 48 degree C, but the normal tissues will not be heated basically. The malignant tumors will be killed selectively, so as to obtain a new strategy for targeted magnetic hyperthermia treatment and prevention with the effective killing of the malignant tumors, and without toxic side effects. Meanwhile, the preparation method for magnetic nanoparticles is developed, with SAR of up to 7000 W/gFe. Animal test results show that targeted nanoparticle drugs for magnetic hyperthermia can cure the maglinant tumors completely without any toxic side effects.

The targeted drugs in this invention are nanoparticle drugs in which antibodies are coupled to magnetic nanoparticles of different particle sizes. Due to the nanometer-scale size, the drug has good penetration, biocompatibility, and long-circulating stability in the blood, allowing it to possess antibody-oriented active targeting function, and magnetic targeting function of magnetism-carried drug and the passive targeting function of nano-drug particles by using EPR effects of tumors. Therefore, it is a composite targeted drug with active targeting function and a variety of other targeting functions.

In the present invention, due to their smaller molecular weight, the antibody Fab fragment and geneticly engineered antibody in the targeted drugs, can penetrate and reach the tumor focus easier than conventional monoclonal antibodies. Magnetic hyperthermia magnetic nanoparticles-antibody targeted drugs comprising antibodies of smaller molecular weight, and higher specificity and affinity possess better penetrating and targeting functions.

The targeted drugs of the invention use magnetic nanoparticles as magnetic hyperthermia thermoseed, antibodies and ligands as guidance molecules, wherein the aforesaid magnetic nanoparticles can concentrate at the tumor focus by drug active targeting and magnetic-targeting at a high concentration via administration routes such as intra-arterial, intravenous or intraperitoneal injection or introduction under the antibody and ligand-oriented targeting and magnetic-targeting reactions. Under the reactions of alternating magnetic field of magnetic hyperthermia therapeutic appratus, the magnetic nanoparticles of the patient's focus can generate significant magnetic loss heat effect, allowing the tumor focus to heat up to 42-95 degree C rapidly, and perform targeted magnetic hyperthermia treatment for 42-48 degree C, or targeted magnetic thermoablation treatment for above 48 degree C.

The application of the magnetic hyperthermia targeted drugs of the present invention for 1 hour or more for one time can effectively kill the malignant tumors of higher affinity with and specificity to antibodies and even can allow the tumors to regress completely. It aslo can effectively kill the malignant tumors of higher affinity with and specificity to antibodies for 1 hour or more for several times, and even can allow the tumors to regress completely. Usually the therapeutic dose of drugs is less than 100 mg.

The targeted drugs of the present invention can be used not only for the targeted hyperthermia treatment on the malignant tumors of higher affinity with and specificity to antibodies or ligands but also for the prevention of malignant tumors of higher affinity with and specificity to antibodies or ligands. They can be used for the active targeting magnetic hyperthermia treatment of detected and undetected malignant tumors, only a very small malignant tumor and the early-stage malignant tumor which cannot be detected by image equipment, the early-stage malignant tumors of only several cells, and the killing treatment of metastatic malignant tumor cell and tumor thrombus, as well as the regular and irregular preventative treatment of active targeting magnetic hyperthermia treatment on the normal persons.

Yet another aspect of the invention is the preparation method of the targeted nanoparticle drugs, comprising coupling of magnetic nanoparticles with guidance molecules at a weight ratio of 1 : 0.0001-0.20 in a solution comprising water, organic substances (such as ethanol, acetate, toluene, or dimethyl sulphoxide), or inorganic substances (such as phosphate), or the mixture thereof. Preferably the above magnetic nanoparticles are treated by the above surfactants and/or surface modifiers at a weight ratio of 1 : 0.0001-0.20, and then are mixed and coupled with the guidance molecules in the above solution.

Yet another aspect of the invention is the use of the above targeted nanoparticle drug in the manufacture of a medicament for treating and preventing tumors.

The magnetic targeted nanoparticle drugs of the present invention have the following characteristics: (1) Magnetic nanoparticle-antibody and ligand active targeting drug is the antibody-mediated or receptor-mediated active targeting magnetic hyperthermia nanoparticle drug using magnetic nanoparticles as the effector molecules, possessing active targeting function, magnetic targeting function of magnetic drugs and passive targeting function of nano-carrier drugs, wherin the aforesaid antibodies or ligands are the guidance molecules of the present invention; (2) Magnetic nanoparticle magnetic targeted drugs are the magnetism-oriented magnetism-targeted magnetic hyperthermia nanoparticle drugs using the magnetic nanoparticles of good *in vivo* biocompatibility as the effector molecules; (3) For the targeted drugs, not only the magnetic nanoparticles of SAR of 10-500 W/gFe are selected, but also those of SAR of as high as 500-7000 W/gFe can be selected. Although the targeted drugs made from 10-500 W/gFe and 500-7000 W/gFe magnetic nanoparticles can be used for the targeted magnetic hyperthermia nanoparticle drugs on tumors, the required amount of magnetic nanoparticles of 10, 500 and 7000 W/gFe for tumor focus is 1.2, 0.024 and 0.0017 g respectively in theory if the targeted drugs are administrated to 5 x 6 x 7 cm encephaloma magnetic hyperthermia treatment requiring 12 W heat power. Considering the densities of iron and iron oxide of 7.9 and 5.25 g/cm³ respectively, the required volumes of the magnetic nanoparticles based on iron and iron oxide in theory are 150, 3, 0.22 µl and 230, 4.6, 0.32 µl respectively for encephaloma magnetic hyperthermia treatment. The targeted nanoparticle drugs made from magnetic nanoparticles of 500-7000 W/gFe will require less magnetic nanoparticles or targeted nanoparticle drugs for treatment, thus having higher clinical performance and greater economic value; (4) The magnetic nanoparticles in the drugs have no toxic side effects to human or animal bodies since they can be degraded in the human or animal bodies. Therefore, they are safe and have no toxic side effects on the targeted magnetic hyperthermia treatment and prevention for malignant tumors; (5) nanometer-scale magnetic nanoparticle antibody/ligand targeted drugs and nano-magnetic particle magnetic targeted drugs have good penetrating abilities, which can allow the drugs to concentrate at the malignant tumor focus by active targeting or magnetic targeting via the administrative routes such as intra-arterial and intravenous injection, intraperitoneal injection, or direct introduction (including: minimal invasion, intervention, injection, etc.), etc.; (6) The magnetic hyperthermia targeted nanoparticle drug for malignant tumors can be applied for the active targeting or magnetic targeting magnetic hyperthermia treatment and prevention of malignant tumors, killing the malignant tumors and cancer cells completely, and offering a new measures of complete cure, safe and non-toxic side effects of targeting magnetic hyperthermia for the treatment and prevention of malignant tumors.

### DETAILED DESCRIPTION OF THE PRESENT INVEION

While the invention is amenable to various modifications and alternative forms, specifics thereof have been shown by way of examples. It should be understood, however, that it is not to limit the invention to the particular embodiments described. On the contrary, the intention is to cover all modifications, equivalents, and alternatives falling within the spirit and scope of the invention as defined by the appended claims.

### Example 1

Fe₂O₃ or Fe₃O₄ or Fe magnetic nanoparticles of particle size distribution range of 1-5 nm, 10-20 nm, 20-40 nm, 50-100 nm, 350-390 nm or 950 nm were added to a sodium oleate aqueous solution at a weight ratio of 1 : 0.0001, 1 : 0.05, 1 : 0.20 between magnetic nanoparticle and sodium oleate respectively, mixed for 10 min ultrasonically, and stirred in water bath at 85 degree C for 8 hours at 600 r/min. After centrifugal separation, the supernatant was discarded, the resultant precipitate was washed fully with deionized water, and then the magnetic nanoparticles modified by sodium oleate were obtained.

### Example 2

Fe₂O₃ or Fe₃O₄ or Fe magnetic nanoparticles of particle size distribution range of 5-10 nm, 50-100 nm, 200-300 nm or 950 nm were added to a decanoic acid aqueous solution at weight ratio of 1 : 0.0001, 1 : 0.01, or 1 : 0.20 between magnetic nanoparticle and decanoic acid respectively, mixed for 10 min ultrasonically, stirred in water bath of 70 degree C for 6 hours at 1000 r/min. After centrifugal separation, the supernatant was discarded, the resultant precipitate was washed fully with deionized water, and then the magnetic nanoparticles modified by decanoic acid were obtained.

### Example 3

Fe₂O₃ or Fe₃O₄ or Fe magnetic nanoparticles of particle size distribution range of 30-50 nm, 40-80 nm, 50-100 nm or 250-350 nm were added to a sodium dodecylsulfate aqueous solution at weight ratio of 1 : 0.0001, 1 : 0.01, or 1 : 0.20 between magnetic nanoparticle and sodium dodecylsulfate respectively, mixed for 10 min ultrasonically, stirred in water bath of 80 degree C for 1 hour at 800 r/min. After centrifugal separation, the supernatant was discarded, the resultant precipitate was washed fully with deionized water, and then themagnetic nanoparticles modified by sodium dodecylsulfate were obtained.

### Example 4

Fe₂O₃ or Fe₃O₄ or Fe magnetic nanoparticles of particle size distribution range of 1-5 nm, 10-20 nm, 20-40 nm, 40-80 nm or 150-200 nm were added to a sodium dodecyl benzene sulfonate aqueous solution at a weight ratio of 1:0.0001, 1:0.01, or 1:0.20 between magnetic nanoparticle and sodium dodecyl benzene sulfonate respectively, mixed for 10 min ultrasonically, stirred in water bath of 85 degree C for 1 hour at 500 r/min. After centrifugal separation, the supernatant was discarded, the resultant precipitate was washed fully with deionized water, and then the magnetic nanoparticles modified by sodium dodecyl benzene sulfonate were obtained.

### Example 5

The modified magnetic nanoparticles in said exampls 1, 2 and 3 were added to a sodium dodecyl benzene sulfonate (SDBS) aqueous solution at a weight ratio of 1 : 0.01, 1 : 0.10, 1 : 0.20 between magnetic nanoparticle and sodium dodecyl benzene sulfonate respectively, mixed for 10 min ultrasonically, stirred in water bath of 85 degree C for 1 hour at 1000 r/min. After centrifugal separation, the supernatant was discarded, the resultant precipitate was washed fully with deionized water, and then the magnetic nanoparticles modified by oleic acid and sodium dodecyl benzene sulfonate, decanoic acid and sodium dodecyl benzene sulfonate, and sodium dodecylsulfate (SDS) and sodium dodecyl benzene sulfonate bimolecular layer were obtained.

### Example 6

Fe₂O₃ or Fe₃O₄ or Fe magnetic nanoparticles of particle size distribution range of 1-5 nm, 10-20 nm, 20-40 nm, 40-80 nm, 350-390 nm or 950 nm were added to a dextran (WM 10000) aqueous solution at a weight ratio of 1 : 0.01, 1 : 0.50, 1 : 1.00, 1 : 2.00, 1 : 5.0 between magnetic nanoparticle and dextran respectively, mixed for 10 min ultrasonically, stirred in water bath of 70 degree C for 1 hour at 1000 r/min. After centrifugal separation, the supernatant was discarded, the resultant precipitate was washed fully with deionized water, and then the magnetic nanoparticles coated by dextran were obtained.

### Example 7

Fe₂O₃ or Fe₃O₄ or Fe magnetic nanoparticles of particle size distribution range of 1-5 nm, 10-20 nm, 20-40 nm, 40-80 nm or 350-390 nm were added to a glucan 40000 or 20000 aqueous solution at weight ratio of 1 : 0.01, 1 : 0.50, 1 : 1.00, 1 : 2.00, or 1 : 5.0 between magnetic nanoparticle and glucan 40000 or 20000 respectively, mixed for 10 min ultrasonically, stirred in water bath of 70 degree C for 1 hour at 1000 r/min. After centrifugal separation, the supernatant was discarded, the resultant precipitate was washed fully with deionized water, and then the magnetic nanoparticles coated by glucan were obtained.

### Example 8

Fe₂O₃ or Fe₃O₄ or Fe magnetic nanoparticles of particle size distribution range of 1-5 nm, 10-20 nm, 20-40 nm, 40-80 nm, 350-390 nm or 950 nm were added to a human serum albumin aqueous soluton at a weight ratio of 1 : 0.05, 1 : 0.20, or 1 : 1.00 between magnetic nanoparticle and human serum albumin respectively, and then mixed for 10 min ultrasonically. After centrifugal separation, the supernatant was discarded, the resultant precipitate was washed fully with deionized water, and then the magnetic nanoparticles modified by human serum albumin were obtained.

### Example 9

Fe₂O₃ or Fe₃O₄ or Fe magnetic nanoparticles of particle size distribution range of 1-5 nm, 10-20 nm, 20-40 nm, 40-80 nm, 350-390 nm or 950 nm wer added to a chitosan aqueous solution at a weight ratio of 1 : 0.05, 1 : 0.20, or 1 : 1.00 between magnetic nanoparticle and chitosan respectively, mixed for 10 min ultrasonically, stirred in water bath of 70 degree C for 1 hour at 800 r/min. After centrifugal separation, the supernatant was discarded, the resultant precipitate was washed fully with deionized water, and then the magnetic nanoparticles coated by chitosan were obtained.

### Example 10

Fe₂O₃ or Fe₃O₄ or Fe magnetic nanoparticles of particle size distribution range of 1-5 nm, 10-20 nm, 20-40 nm, 40-80 nm, or 350-390 nm were added to a polyethylene glycol aqueous solution at a weight ratio of 1:0.05, 1:0.20, 1:0.50, or 1:1.00 between magnetic nanoparticle and polyethylene glycol 2000, polyethylene glycol 4000, polyethylene glycol 6000 or polyethylene glycol 20000 respectively, mixed for 10 min ultrasonically, stirred in water bath of 80 degree C for 10 hours at 500 r/min. After centrifugal separation, the supernatant was discarded, the resultant precipitate was washed fully with deionized water, and then the magnetic nanoparticles modified by polyethylene glycol were obtained.

### Example 11

An ethanol solution at 2:1 molar ratio between soybean lecithin and cholesterol was prepared, then quickly injected with a syringe into a phosphate buffered saline containing 1-5 nm, 10-20 nm, 20-40 nm, 40-80 nm or 350-390 nm of Fe₂O₃ or Fe₃O₄ or Fe magnetic nanoparticles respectively, stirred for 30 minutes at 300, 400, 600 rpm at the temperature of 40 degree C respectively. After ethanol was volatilized, then the unwrapped magnetic particles and the magnetic particles unwrapped by liposomes were discarded by centrifugation, and thus the liposome-wrapped magentic nanoparticles were obtained. If the magnetic nanoparticles with particle size of less than 40 nm were selected, then the liposome-wrapped magentic nanoparticle of an average particle size of less than 50 nm can be obtained.

### Example 12

20-40 nm magnetic nanoparticles were added into 400 ml of ethanol/water solution at a weight ratio of 1:1 between ethanol and water, followed by 10 minutes of powerful ultrasonic dispersion, and addition of 2% of volume ratio of γ-methyl acryloyloxypropyl trimethoxy silane (CH₂C(CH₃)C(O)OC₃H₆Si(OCH₃)₃, KH-570 silicon coupling agent). The system should be kept closed to avoid solvent evaporation. After strong agitation for 5 hours at 50 degree C, and magnetic separation after cooling, the magnetic nanoparticles were washed with ethanol and then deionized water, followed by addition of nano-SiO₂ at a weight ratio of 5.0, 1.0, 0.5, or 0.1 : 1 between nano-SiO₂ and magnetic nanoparticle. After 10 minutes of powerful ultrasonic dispersion and mixing, magnetic nanoparticles were separated by magnetic separation and washed three times with anhydrous ethanol. After the supernatant was discarded, 1% PBS was added into the resultant precipitate, and the magnetic nanoparticles coated with different thickness of SiO₂ were obtained.

### Example 13

Into 2.5 ml of 0.6 % HAuCl₄ aqueous solution, 120 ml of double-distilled water was added, pH adjusted to 7.2 with 0.2 mol/L K₂CO₃, followed by addition of gold and magnetic nanoparticles at a weight ratio of 5, 3, 0.01 : 1 between gold and magnetic nanoparticles, then 1 ml of white phosphorus ethyl ether solution (1 part of the saturated white phosphorus ethyl ether solution added with 4 parts of ethyl ether). After 15 minutes of shaking at room temperature, the solution turns into reddish-brown, and is then heated to wine red. Finally magnetic nanoparticles coated with different thickness of nano-gold were obtained.

### Example 14

The magnetic nanoparticles coated by liposomes in said example 11 were mixed with an equal volume of PBS solution containing 2% PEG5000, and settled for 60 min at 10 degree C, followed by addition of the same volume ofPBS solution and stirring for 1 hour at 800 r/min at 10 degree C. The supernatant was discarded after centrifugal separation and the resultant precipitate was fully washed with deionized water, and the magnetic nanoparticles coated by liposomes and modified by PEG with good long circulating stability *in vivo* were obtained.

### Example 15

The aqueous solution of magnetic nanoparticles coated by dextran containging 20 mg of iron in said example 6 was mixed with 500 mg NaIO₄, oxidized for 60 minutes at room temperature while protected from exposure to light and then dialyzed for 20 hours against 1000 ml of 20 mM sodium borate (pH8.5). 1 /10 of the mixture was mixed with 1.0 mg monoclonal antibody HAb18 in 0.5 mL of 20 mM NaIO₄ buffer at room temperature for 4 hours, and reduced by 50 µl 0.25 M NaBH₄ for 30 minutes at 4 degree C. Then dextran glucose group on the magnetic nanoparticle surface was converted into aldehyde group and then covalently bound to the amino group of monoclonal antibody molecules. After magnetic separation, the magnetic nanoparticles were washed with deionized water, the supernatant was discarded, and 1% of PBS was added. Finally the targeted nanoparticle drug of magnetic nanoparticles - anti-human hepatoma McAb HAb18 was obtained.

### Example 16

The aqueous solution of magnetic nanoparticles coated by glycan containing 20 mg of iron obtained from said example 7 was mixed with 500 mg NaIO₄, oxidized for 60 minutes at room temperature while prevented from exposure to light, dialyzed for 20 hours against 1000 ml of 20 mM sodium borate (pH8.5). 1 /10 of the mixture was mixed with 0.25 mg monoclonal antibody HAb18 Fragment HAb18F (ab')₂ in 0.5 ml of 20 mM NaIO₄ buffer solution at room temperature for 4 hours, then reducted by 50 µl of 0.25M NaBH₄ for 30 minutes at 4 degree C. After magnetic separation and washing with 1% of PBS, the targeted nanoparticle drug of magnetic nanoparticle - anti-human hepatoma McAb HAb 18 fragment HAb 18 F (ab')₂ was obtained.

### Example 17

The magnetic nanoparticles were added into 400 ml of ethanol /water solution at a weight ratio of 1:1 between ethanol and water, followed by 10 minutes of powerful ultrasonic dispersion and addition of 2% volume ratio of γ-methyl acryloyloxypropyl trimethoxy silane (CH₂C(CH₃)C(O)OC₃H₆Si(OCH₃)₃, KH-570 silicon coupling agent). The system was kept closed to avoid solvent evaporation. After strong agitation for 5 hours at 50 degree C, the magnetic nanoparticles were separated by magnetic separation after cooling, washed with ethanol and then with deionized water, and then added with ABX-EGF antibody at weight ratio of 20 : 0.01, 1, 5, or 10 between magnetic particle and antibody. After 10 minutes of powerful ultrasonic dispersion and mixing, magnetic nanoparticles were separated by magnetic separation, washed three times with deionized water. The supernatant was discarded and 1% PBS was added. Finally the targeted nanoparticle drug of magnetic nanoparticles - ABX-EGF anbody was obtained.

### Example 18

3 mg anti-CEA antibodies after PBS dialysis overnight were adjusted to 2 ml in volume, added with 10 µl of 20 mmol/l heterotypic dual function crosslinking agent N-succinimidyl-3-(2-pyridyldithio)propionate (SPDP) prepared in anhydrous ethanol, stirred slowly for 60 minutes at room temperature and passed through Sephadex G25 chromatography column and eluted with PBS. The first peak was collected and concentrated into 2 ml. After the sterilization by filtration, purified anti-CEA antibody-PDP was obtained. The aforesaid magnetic nanoparticle coated by human serum albumin in said example 8 through ⁶⁰Co radiation sterilization treatment containing 30 mg iron was dispersed into 3 ml distilled water homogeneously, added with 10-20 µl SPDP and stirred for 60 minutes. Magnetic particle coated by PDP was obtained after remaining SPDP was removed by centrifugation. The magnetic nanoparticle coated by PDP was dispersed in 3 ml of 0.05 mmol/l acetate buffer solution of pH 4.5 under ultrasound, added with dithiothreitol (DTT) up to a concentration of 50 mmol/l, stirred for 30 minutes at room temperature for reduced human serum albumin - PDP. The remaining DTT was removed by centrifugation. The precipitate was washed with PBS and then human serum albumin - PDP-SH sulfhydryl was obtained. It was then suspended into 2 ml PBS, mixed with the anti-CEA antibody - PDP immediately, stirred for 24 hours at room temperature or dispersed for 10 minutes under powerful ultrasonic. After centrifugation, the precipitate was washed with 1% normal saline and the targeted nano-particle drug of magnetic nanoparticle - anti-CEA antibody was obtained.

### Example 19

The aqueous solution of magnetic nanoparticles coated with chitosan containing 10 mg iron obtained in said example 9 were dripped into a toluene solution containing diethyl octyl sodium sulfosuccinate surfactant, and mechanically agitated to achieve an emulsion. 25% glutaraldehyde solution was dripped to the emulsion and reacted under mechanical agitation for 1 hour at 2000 r/min. After reaction, the surfactant and organic solvent were removed by washing repeatedly with ethanol, acetone and distilled water. Magnetic particle were separated with magnetic separator, washed and dispersed in distilled water at 4 degree C, and the magnetic nanoparticles coated by chitosan whose surface contained rich aldehyde group were obtained. These magnetic nanoparticles were dispersed into 5% glutaraldehyde solution, shaked for 2 hours at room temperature, and then washed with PBS buffer solution, followed by addition of anti-HER2 antibody based on magnetic particle and antibody at a weight ratio of 20 : 0.01, 0.1, 1.0, or 10. After reaction for 12 hours at 4 degree C, washing for three times with PBS and 0.2% NaN₃ in 1% PBS buffer solution, respectively, and dispersing in 2 ml PBS, the targeted nanoparticle drug of magnetic nanoparticle - anti-HER2 antibody was obtained.

### Example 20

The magetic nanoparticles of particle size range of 1-5 nm, 10-20 nm, 20-40 nm, 50-100 nm or 950 nm (SAR of 501-7000 W/gFe) were dispersed into deionized water for 10 minutes under strong ultrasound respectively, and added with Rituxan antibody at a weight ratio of 0.001, 0.1, 1, 2 : 10 between antibody and magentic nanoparticles respectively. It was then quickly stirred and mixed, dispersed for 10 minutes under powerful ultrasound and added with 1% BPS. After centrifugation and washing three times with 1% PBS, the targeted nanoparticle drug of magnetic nanoparticle - Rituxan antibody was obtained.

### Example 21

The magnetic nanoparticles modified by a surfactant prepared in said example 1-5 were dispersed into deionized water for 10 minutes under powerful ultrasound respectively, added with the CEA antibody at a weight ratio of 0.001, 0.1, 1, 2 : 10 between antibody and magnetic nanoparticle, quickly stirred and mixed, dispersed for 10 minutes under powerful ultrasound, and added with 1% BPS. After centrifugation and washing three times with 1% PBS, the targeted nanoparticle drug of magnetic nanopariticle - anti-CEA antibody was obtained.

### Example 22

The magnetic nanoparticles containing 30 mg iron coated by human serum albumin obtained in said example 8 through ⁶⁰Co radiation sterilization treatment were dispersed into 3 ml of deionized water homogenously, added with 10-20 µl SPDP, and stirred for 60 minutes. After the remaining SPDP was removed by centrifugation, magnetic nanoparticles coated by PDP were obtained. 0.1, 1.0, 5.0, 10 mg transferrin was dissolved into PBS to prepare a solution with a weight ratio of 0.1-50%, to which the magnetic nanoparticles coupled with PDP was quickly added at -20 degree C to 30 degree C and at 800-1200 r/min, and then dispersed under powerful ultrasound and mixed. After magnetic separation and addition of 1% normal saline to wash for three times, targeted nanoparticle drug of magnetic nanoparticle - transferrin was obtained.

### Example 23

The magnetic nanoparticles modified by polyethylene glycol in said example 10 was added quickly at -20 degree C to 30 degree C and at 800-1200 r/min into a solution, which is prepared by dissolving 0.1, 1.0, 5.0, 10 mg transferrin into phosphate buffered saline to prepare the solution at a weigh ratio of 0.1-50%. After powerful ultrasonic dispersion and mixing, magnetic nanoparticles were separated and washed three times with 1% PBS, the targeted nanoparticle drug of magnetic nanoparticle - transferrin was obtained.

### Example 24

13.2 mg 2-deamino hydroxy folate FA was dissolved into 1 mL of dimethyl sulfoxide (DMSO), and a certain mount of dicyclohexylcarbodiimide (DCC) and N-Hydroxysuccinimide (NHS) was added under stirring condition. Then 100 mg of polyethylene glycol di-amine (PEG-NH₂) was added and allowed for reaction for 4 hours at room temperature, and added 5 ml of deionized water. The insoluble substances were removed by filtration, and the supernatant was freeze-dried. After washing and drying with ethyl ether, yellowish solid FA-PEG-NH₂ was obtained. 10 or 100 mg magnetic nanoparticles coated by chitosan in said example 9 were dissolved into 1 ml deionized water, dispersed ultrasonically for 10 minutes, added with 0.1 mg FA-PEG-NH₂. After coupling through amino to the nanoparticles and coincubating 3 hours at room temperature, the magnetic nanoparticles were separated by magnetic separation. The supernatant was discarded, the normal saline was added, and then the targeted nanoparticle drug of magnetic nanoparticle - folate was obtained.

### Example 25

13.2 mg 5-methyl tetrahydrofolic acid (THFA) FA was dissolved into 1 ml of dimethyl sulfoxide (DMSO), and a certain mount of dicyclohexylcarbodiimide (DCC) and N-Hydroxysuccinimide (NHS) was added under stirring condition. Then 100 mg of polyethylene glycol di-amine (PEG-NH₂) was added and allowed for reaction for 4 hours at room temperature, followed by addion of 5 ml of deionized water. The insoluble substances were removed by filtration, and the supernatant was freeze-dried. After washing and drying with ethyl ether, yellowish solid FA-PEG-NH₂ was obtained. 10 or 100 mg magnetic nanoparticles coated by glucan in said example 7 was dissolved into 1 ml deionized water, dispersed ultrasonically for 10 minutes, added with 0.1 mg FA-PEG-NH₂. After coupling through amino to the nanoparticles and coincubating 3 hours at room temperature, magnetic nanoparticles were separated by magnetic separation. The supernatant and the magnetic nanoparticle uncoated with folate were discarded, 1% PBS was added, and then the targeted nanoparticle drug of magnetic nanoparticle - folate was obtained.

### Example 26

Appropriate amount of dicyclohexylcarbodiimide (DCC) and N-hydroxysuccinimide (NHS) were added under stirring condition to 50 mg of folic acid dissolved in 1 ml of dimethyl sulfoxide (and 0.025 ml of triethylamine, TEA), and held for reaction at room temperature overnight. After by-product dicyclohexylurea was discarded by filtration, yellowish powder of folic acid active ester was obtained after concentation and drying under reduced pressure. The colloid aqua of magnetic nanoparticles coated by human serum albumin containing 100, 200 or 500 mg of Fe in said example 8 were adjusted to pH 9, added with proper amount of dimethyl sulfoxide solution of folic acid active ester, and stirred at room temperature. Magnetic nanoparticles were then separated by magnetic separation, washed with 1% normal saline. Finally the targeted nanoparticle drug of magnetic nanoparticle - folate was obtained.

### Example 27

Diethylenetriamine pentaacetic acid - Folate (DTPA-Folate) was synthesized according to Mathias's method (see Nuclear Medicine and Biology, 25:585-587 (1998)). 10 mg of DTPA-Folate were dissolved into 1.5 ml water, added with 100 mg of magnetic nanoparticle with size distribution of 1-5 nm, 5-10 nm, 10-20 nm, 20-40 nm, or 50-100 nm, dispersed for 10 minutes under powerful ultrasound respectively, stirred for 1 hour at room temperature under 400 r /min. Magnetic nanoparticles were then separated by magnetic separation, washed three times with 1% PBS, added with 1% PBS and finally the targeted nanoparticle drug of magnetic nanoparticle - folate was obtained.

The drugs made from above samples were tested for particle size under conventional testing methods, and the particle size is 2-1000 nm.

### Example 28

16 nude mice for subcutaneous-inoculation of colon cancer cells LS 174T were selected, half-male and half-female, randomly divided into 2 groups according to the tumor volume after measuring and calculating the tumor volume when the tumor size was about 5-10 mm. 0.2 ml deionized water solution of the aforesaid glucan coated magnetic nanoparticle (with 300-400 nm particle size) containing 40 mg of iron prepared in said example 7 was administrated to each nude mouse in the two groups through intravenous injection. 0.6T *in vitro* magnetic field was exerted on tumor positions of one group for 30 minutes. The magnetic particle content of tumor positions exerted *in vitro* magnetic field was about 7 times higher than that of tumor positions without magnetic field, based on the measurement results of phenanthridines determination method of ferrous ion.

### Example 29

16 nude mice for the subcutaneous-inoculation of colon cancer cells LS 174T were selected, half-male and half-female, randomly divided into 2 groups according to the tumor volume after measuring and calculating the tumor volume when the tumor size was about 5-10 mm. 0.2 ml deionized water solution of the aforesaid polyethylene glycol modified magnetic nanoparticle of long circulating stability *in vivo* (with about 3-20 nm particle size) containing 40 mg of iron prepared in said example 10 was administrated to each nude mouse in the two groups through intravenous injection. 0.8T *in vitro* magnetic field was exerted on tumor positions of one group for 30 minutes. For the tumor positions on which *in vitro* magnetic field was exerted, 50% or more magnetic nanoparticles were gathered in the tumor positions, based on the measurement results of phenanthridines determination method of ferrous ion.

### Example 30

16 nude mice for subcutaneous-inoculation of colon cancer cells LS174T were selected, half-male and half-female, randomly divided into 2 groups according to the tumor volume after measuring and calculating the tumor volume when the tumor size was about 5-10 mm. 0.2 ml deionized water solution of the aforesaid liposomes coated and PEG modified magnetic nanoparticles with long circulating stability *in vivo* (with 50-100 nm particle size) containing 40 mg of iron prepared in said example 14 was administrated to each nude mouse in the two groups through intravenous injection. 0.6T *in vitro* magnetic field was exerted on tumor positions of one group for 30 minutes. The magnetic particle content of tumor positions on which magnetic field was exerted was about 9 times higher than that of tumor positions without magnetic field, based on the measurement results of phenanthridines determination method of ferrous ion.

### Example 31

16 nude mice for subcutaneous-inoculation of hepatoma cells HHCC were selected, half-male and half-female, randomly divided into 2 groups according to the tumor volume after measuring and calculating the tumor volume when the tumor was about 5-10 mm. 0.2 ml of the 1% PBS solution of the aforesaid magentic nanoparticle - anti human hepatoma monoclonal antibody HAb18 targeted nanoparticle drugs (with about 20-40 nm particle size) containing 30 mg of iron prepared in said example 15 was administrated to each nude mouse in the two groups through intravenous injection. 0.3T *in vitro* magnetic field is exerted on one group for 60 minutes. The magnetic nanoparticle content of tumor positions no which *in vitro* magnetic field was exerted was about 138 times higher than that of tumor positions without magnetic field, based on the measurement results of phenanthridines determination method of ferrous ion.

### Example 32

16 kunming mice for subcutaneous-inoculation of sarcoma S-180 were selected, half-male and half-female, randomly divided into 2 groups according to the tumor volume after measuring and calculating the tumor volume when the tumor size was about 5-10 mm. 0.2 ml of the 1% PBS solution of the aforesaid magentic nanoparticle - transferrin targeted nanoparticle drugs (with about 10-30 nm particle size) containing 40 mg of iron prepared in said example 23, was administrated to each mouse in the two groups through intravenous injection. 0.3T *in vitro* magnetic field was exerted on tumor positions of one group for 30 minutes. The magnetic particle content of tumor positions on which magnetic field was exerted was about 43 times higher than that of tumor positions without magnetic field, based on the measurement results of phenanthridines determination method of ferrous ion.

### Example 33

16 nude mice for subcutaneous-inoculation of liver cancer cells BEL7404 were selected, half-male and half-female, randomly divided into 2 groups according to the tumor volume after measuring and calculating the tumor volume when the tumor size was about 5-10 mm. 0.2 ml of the normal saline solution of the aforesaid magentic nanoparticle - folate targeted nanoparticle drugs (with about 10-30 nm particle size) containing 40 mg of iron prepared in said example 26, was administrated to each nude mouse in the two groups through intravenous injection. 0.3T *in vitro* magnetic field was exerted on tumor positions of one group for 30 minutes. The magnetic particle content of tumor positions on which magnetic field was exerted was about 31 times higher than that of tumor positions without magnetic field, based on the measurement results of phenanthridines determination method of ferrous ion.

### Example 34

Zetasizer 3000 laser particle-size analyzer and JEM-2010 UHR high resolution transmission electron microscope were used to observe the particle size and surface change of modified, wrapped or coated magnetic nanoparticles, and magnetic nanoparticle-antibody or -ligand conjugates prepared in aforesaid examples. It was found that (1) surfactant layer is usually less than 1 nm for said samples in examples 1, 2, 3 and 4, and about 1 nm for said samples in example 5; (2) 10-20 nm of organic substance layer can be formed in said samples in examples 6, 7, 8, 9, 10 and 14, and 8-10 nm of liposome layer in example 11. Inorganic substance layer of about 5-50 nm is formed in examples 12 and 13; (3) The particle sizes of magnetic nanoparticle-antibody conjugates in examples 15, 16, 17, 18, 19, 20 and 21 can be increased by only about 6-20 nm than those of modified magnetic nanoparticles and coated magnetic nanoparticles in said examples and those of bare magnetic nanoparticles; (4) The particle sizes of magnetic nanoparticle - transferrin conjugates in the above examples 22 and 23 can be increased by only 8-20 nm as compared to those of modified magnetic nanoparticles and coated magetic nanoparticles prior to coupling and those of bare magnetic nanoparticles; (5) The particle sizes of magnetic nanoparticle - folate conjugates in the above examples 24, 25, 26 and 27 can be increased by only about 5-10 nm as compared to those of modified magnetic nanoparticles and coated magetic nanoparticles prior to coupling and those of bare magnetic nanoparticles. The above magnetic nanoparticle-antibody targeted nanoparticle drugs, and magnetic nanoparticle - ligand targeted nanoparticle drugs made from the nanoparticles of 1-40 nm have a good dispersibility and a dispersion stability for several months to four years or more.

### Example 35

Analysis by JEM-2010 UHR high resolution transmission electron microscope demonstrated that the targeted nanoparticle drugs (conjugates) of superparamagnetic nanoparticle with particle size of 10 nm and anti-CEA-antibody, anti-human hepatoma McAb HAb18 fragment HAb18 F (ab')₂ or anti-HER2 antibody in aforesaid examples have particle sizes of about 20 nm. The magnetic nanoparticles in the drugs can form good coupling with the antibodies, with at lease one antibody coupled to surface of a magnetic nanoparticle.

### Example 36

For long period of storage and convenient use, the above targeted nanoparticle drugs, modified magetic nanoparticle and coated or wrapped magetic nanoparticle prepared from above examples can be made by freeze-drying into solid freeze-drying powder. In practical use, the targeted drugs freeze-drying powder can be made with the deionized water, 1% PBS solution, 1% normal saline solution or a nucleotide aqueous solution (for injection formulation, the weight ratio of the deionized water to nucleotide in the solvent is 1: 0.20) into the targeted nanoparticle drug injection in which the weight ratio of the targed drugs to the deionized water, PBS solution, normal saline solution or nucleotide aqueous solution was 1: ≤ 0.5, which can be immediately used. Similarly, the aforesaid modified magetic nanoparticle and coated or wrapped magetic nanoparticle freeze-drying powder can be added into proper amount of water for further processing use. Usually, the targeted drug prepared in above examples can be directly made into the targeted nanoparticle drug injection formulation, in which the weight ratio of the targeted drugs to the deionized water, PBS solution, normal saline solution or nucleotide aqueous solution was 1:≤ 0.5.

### Example 37

(1) The immunocompetence of magentic nanoparticle - anti-CEA antibody targeted nanoparticle drug with 20-70 nm of particle size was measured by ELISA (enzyme-linked immunosorbent assay) experiment, with 63% acheived.
(2) The *in vitro* stability experiment from the injections of the above traged drugs showed that shedding rate of magnetic nanoparticles is less than 5% after 24 hours of incubation at 37 degree C, either in a normal saline solution or in human serum albumin. The magentic nanoparticle - anti-CEA antibody targeted nanoparticle drug was approved to be stable *in vitro.*

### Example 38

(1) The immunocompetence of magentic nanoparticle - anti-human hepatoma McAb HAb18 targeted nanoparticle drug with 40-80 nm of particle size was measured by ELISA experiment, with 57% acheived. (2) The *in vitro* stability experiment from the injections of the above traged drugs showed that shedding rate of magnetic nanoparticles is less than 5% after 24 hours of incubation at 37 degree C, either in a normal saline solution or in human serum albumin. The magentic nanoparticle - anti-human hepatoma McAb HAb18 targeted nanoparticle drug was approved to be stable *in vitro.*

### Example 39

(1) Ferromagnetic nanoparticle - anti-CEA antibody targeted nanoparticle drug with 40-80 nm of particle size was mixed with colon cancer cell LS174T. Tested on Zetasizer 2000 laser particle-size analyzer under ultrasonic power of 1-25 W, magnetic nano-particle - anti-CEA antibody targeted nanoparticle drug and magnetic nanoparticles can bind with cancer cells and antibodies respectively and show no falling out. (2) Ferrimagnetic nanoparticle - anti-CEA antibody targeted nanoparticle drug with 40-80 nm of particle size was mixed with colon cancer cells LS174T, blood cells and normal liver cells. Only colon cancer cell LS174T was retained under the magnetic field after magnetic separation, and blood cells and normal liver cells were completely separated from colon cancer cell LS174T. The results showed that, magnetic nanoparticle and anti-CEA antibody had powerful coupling bonding force and stability. The magnetic nano-particle - anti CEA antibody targeted nanoparticle drug had good targeting performance and powerful affinity towards colon cancer cell LS174T, and also showed good biocompatibility with blood, liver, etc.

### Example 40

(1) Ferromagnetic nanoparticle - anti-human hepatoma McAb HAb18 targeted nanoparticle drug of 20-70 nm of particle size was mixed with human liver cancer cell HHCC. Tested on Zetasizer 2000 laser particle-size analyzer under ultrasonic power of 1-25 W, magnetic nanoparticle - anti-human hepatoma McAb HAb18 targeted nanoparticle drug and magnetic nanoparticles can bind with cancer cells and antibodies respectively and show no falling out. (2) Magnetic nanoparticle - anti-human hepatoma McAb HAb18 targeted nanoparticle drug of 20-70 nm particle size was mixed with human liver cancer cell HHCC, blood cells and normal liver cells, only human liver cancer cell HHCC was retained under the magnetic field after magnetic separation, and blood cells and normal liver cells were completely separated from liver cancer cell HHCC. The results showed that, magnetic nanoparticle - anti-human hepatoma McAb HAb18 had powerful coupling bonding force and stability. The magnetic nanoparticle - anti-human hepatoma McAb HAb18 targeted nanoparticle drug had a good targeting performance and powerful affinity towards liver cancer cell HHCC, and also showed good biocompatibility with blood, liver, etc.

### Example 41

Nude mice with subcutaneous colon cancer cell LS174T tumors were injected with 30 mg of superparamagnetic nanoparticle - anti-CEA antibody targeted nanoparticle drug with 20-50 nm of particle size through caudal intravenous injection. After 30 minutes, the nude mice were killed, and tumors and other vital organs such as heart, brain, spleen, kidney, intestine, liver, etc, were taken for scanning electron microscopic study. The results of the electron microscopic analysis and energy spectrum analysis showed that the weight percentage of Fe element among 10 kinds of major elements (Fe, Ca, Si, Al, Mg, K, Na, P, O, C etc) in the tumor capillary vessel blood and in the tumor tissue of the nude mice, which were injected by targeted nanoparticle drugs (Fe is a main element of the magnetic nanoparticle and accounts for 70% of the magnetic nanoparticule based on weigh), was increased to 23.11% and 0.83% respectively, from 0.08% and 0% in the tumor capillary vessel blood and tumor tissue of nude mice in the control group (no targeted drug is injected), and also higher than that in the non-tumor blood vessel of the nude mice which were injected with targetd nanoparticle drug (0.14%), that in the normal liver tissue (0.14%), and that in other organs (less than 0.14%). Targeted nanoparticle drug contents in the tumor capillary vessel blood and tumor tissue of the nude mice which were injected by targeted nanoparticle drugs were at least 289 times (because Fe can't be detected in the tumors of control group, it is unable to calculate), and 165 times and 165 times higher than that in the tumor capillary vessel blood and tumor tissue of nude mice in the control group (no targeted drug is injected), that in the non-tumor blood vessel of nude mice which were injected with targetd nanoparticle drug and that in the normal liver tissue, that was, targeted nanoparticle drug content in tumor was at least 165 times higher than that in other positions. The magnetic nanoparticle - anti-CEA antibody targeted nanoparticle drug is of significant targeting performance *in vivo,* and enable targeted nanoparticle drug to concentrate in the tumor focus, but doesn't concentrate in other positions.

### Example 42

Nude mice with subcutaneous liver cancer cell HHCC tumors were injected with 30 mg of superparamagnetic nanoparticle - anti-human hepatoma McAb HAb18 targeted nanoparticle drug with 20-50 nm of particle size through caudal intravenous injection. After 30 minutes, the nude mice were killed, and tumors and other vital organs such as heart, brain, spleen, kidney, intestine, liver, etc, were taken for scanning electron microscopic study.

The results of electron microscopic analysis and energy spectrum analysis, showed that the weight percentage of Fe element among 10 kinds of major elements (Fe, Ca, Si, Al, Mg, K, Na, P, O, C etc) in the tumor capillary vessel blood and tumor tissue of nude mice, which were injected by targeted nanoparticle drugs (Fe is a main element of the magnetic nanoparticle and accounts for 70% of magnetic nanoparticules based on weight), was increased to 24.38% and 0.76% respectively, from 0.08% and 0% in the tumor capillary vessel blood and tumor tissue of nude mice in the control group (no targeted drug is injected), and also higher than that in the non-tumor blood vessel of the nude mice which were injected with targetd nanoparticle drug (0.14%), that in the normal liver tissue (0.14%), and that in other organs (less than 0.14%). Targeted nanoparticle drug contents in the tumor capillary vessel blood and tumor tissue of the nude mice which were injected by targeted nanoparticle drugs were at least 304 times (because Fe can't be detected in the tumors of control group, it is unable to calculate), and 174 times and 174 times higher than that in the tumor capillary vessel blood and tumor tissue of nude mice in the control group (no targeted drug is injected), that in the non-tumor blood vessel of nude mice which were injected with targetd nanoparticle drug and that in the normal liver tissue, that was, targeted nanoparticle drug content in tumor was at least 174 times higher than that in other positions. The magnetic nanoparticle - anti-CEA antibody targeted nanoparticle drug is of significant targeting performance *in vivo,* and enable targeted nanoparticle drug to concentrate in the tumor focus, but doesn't concentrate in other positions.

### Example 43

16 nude mice for subcutaneous-inoculation of human gastric cancer cell N87 were selected, half-male and half-female, randomly divided into 2 groups based on the tumor volume when the tumor size was about 5-10 mm after measuring and calculating the tumor volume. The first group was the control group, where each nude mouse was injected with 0.3 ml of deionized water. Each nude mouse in the second group was injected intraperitoneally with 0.3 ml injection formulation containing 30 mg superparamagnetic iron oxide nanoparticle (with SAR of 1000 W/gFe) - anti-HER2 antibody targeted nanoparticle drug with 30-50 nm particle size prepared, and then placed in the alternating magnetic field of magnetic hyperthermia therapeutic apparatus to raise the temperature of tumor to 42-43 degree C for treatment of one hour twice. After treatment for 10 days, the aforesaid nude mice were killed, and the tumors were separated and weighed. The results showed that the tumor weight of was 2.36 ± 0.53 g, 0.97 ±0.29 g, for the first and the second group, respectively. The tumor inhibition rate in the therapeutic group was 59%, with a P value less than 0.01.

### Example 44

16 nude mice for subcutaneous-inoculation of human liver cancer cell HHCC were selected, half-male and half-female, randomly divided into 2 groups based on the tumor volume when the tumor size was about 5-10 mm after measuring and calculating the tumor volume. The first group was the control group, where each nude mouse was injected with 0.3 ml of deionized water. Each nude mouse in the second group was injected intraperitoneally with 0.3 ml of injection formulation containing 30 mg superparamagnetic iron oxide nanoparticle (with SAR of 2000 W/gFe) - anti-human hepatoma McAb HAb18 targeted nanoparticl drug with 20-40 nm particle size prepared, and then placed in the alternating magnetic field of magnetic hyperthermia therapeutic apparatus to raise the temperature of tumor to 42-43 degree C for treatment of 65 minutes twice. After treatment for 10 days, the aforesaid nude mice were killed, and the tumors were separated and weighed. The results showed that the tumor weight of was 2.97 ± 0.65 g, 1.02 ± 0.31 g, for the first and the second group, respectively. The tumor inhibition rate in the therapeutic group was 66%, with a P value less than 0.01.

### Example 45

16 nude mice for subcutaneous-inoculation of human lung cancer cell A549 were selected, half-male and half-female, randomly divided into 2 groups based on the tumor volume when the tumor size was about 5-10 mm after measuring and calculating the tumor volume. The first group was the control group, where each nude mouse was injected with 0.3 ml of deionized water. Each nude mouse in the second group was injected intraperitoneally with 0.3 ml of injection formulation containing 30 mg iron oxide superparamagnetic nanoparticle (with SAR of 200 W/gFe) - anti-CEA antibody targeted nanoparticle drug with 50-100 nm particle size prepared, and then placed in the alternating magnetic field of magnetic hyperthermia therapeutic apparatus to raise the temperature of tumor to 42-43 degree C for treatment of 65 minutes twice. After treatment for 10 days, the aforesaid nude mice were killed, and the tumors were separated and weighed. The results showed that the tumor weight of was 1.97 ± 0.53 g, 0.79 ± 0.37 g, for the first and the second group, respectively. The tumor inhibition rate in the therapeutic group was 60%, with a P value less than 0.01.

### Example 46

16 nude mice for subcutaneous-inoculation of colon cancer cell LS174T were selected, half-male and half-female, randomly divided into 2 groups based on the tumor volume when the tumor size was about 5-10 mm after measuring and calculating the tumor volume. The first group was the control group, where each nude mouse was injected with 0.3ml of deionized water. Each nude mouse in the second group was injected through caudal intravenous injection with 0.3 ml injection formulation of magnetic nanoparticle (with SAR of 300 W/gFe) - anti-CEA antibody targeted nanoparticle drug with 40-80 nm particle size containing 50 mg of Fe prepared, and then placed in the alternating magnetic field of magnetic hyperthermia therapeutic apparatus to raise the temperature of tumor to 42-43 degree C for treatment of 1 hour once. After treatment for 10 days, the aforesaid nude mice were killed, and the tumors were separated and weighed. The results showed that the tumor weight of was 2.97 ± 0.65 g, 0.12 ± 0.31 g, for the first and the second group, respectively. The tumor inhibition rate in the therapeutic group was 96%, with a P value less than 0.01. Among them two nude mice had their tumors completely eliminated and regressed. Based on the tumor remaining tissue pathology photograph analysis, complete coagulation necrosis was observed on the tumor cells on three nude mice, only a few of tumor cells with non-necrosis were observed on two nude mice. After treatment for 10 days, no obvious weight loss and adverse reaction of the nude mice in the second group were observed and measured. After the nude mice were killed, the vital organs such as heart, brain, spleen, kidney, intestine, liver, etc, were taken for pathological examination and no obvious abnormality was found. Animal tests showed that the magnetic hyperthermia of the magentic nanoparticle - anti-CEA antibody targeted nanoparticle drug can kill the tumors targetedly with high-effectivity, safety and no toxic side effects. The tumors can even be eliminated and regressd completely.

### Example 47

16 nude mice for subcutaneous-inoculation of human liver cancer cell HHCC were selected, half-male and half-female, randomly divided into 2 groups based on the tumor volume when the tumor size was about 5-10 mm after measuring and calculating the tumor volume. The first group was the control group, where each nude mouse was injected with 0.3 ml of deionized water. Each nude mouse in the second group was injected through caudal intravenous injection with 0.3 ml injection formulation of magnetic nanoparticle (with SAR of 300 W/gFe) - anti-human hepatoma McAb HAb18 fragment HAb18F (ab')₂ targeted nanoparticle drug with 40-80 nm particle size containing 50 mg of Fe prepared, and then placed in the alternating magnetic field of magnetic hyperthermia therapeutic apparatus to raise the temperature of tumor to 42-43 degree C for treatment of 1 hour once. After treatment for 10 days, the aforesaid nude mice were killed, and the tumors were separated and weighed. The results showed that the tumor weight was 2.97 ± 0.65 g, 0.12 ± 0.31 g, for the first and the second group, respectively. The tumor inhibition rate in the therapeutic group was 96%, with a P value less than 0.01. Among them two nude mice had their tumors completely eliminated and regressed. Based on the tumor remaining tissue pathology photograph analysis, complete coagulation necrosis was observed on the tumor cells on two nude mice, and only a few of tumor cells with non-necrosis were observed on three nude mice. After treatment for 10 days, no obvious weight loss and adverse reaction of the second nude mice were observed and measured. After the nude mice were killed, the vital organs such as heart, brain, spleen, kidney, intestine, liver, etc, were taken for pathological examination and no obvious abnormality was found. Animal tests showed that the magnetic hyperthermia of the magentic nanoparticle - anti-human hepatoma McAb HAb18 fragment HAb18F (ab')₂ targeted nanoparticle drug can kill the tumors targetedly with high-effectivity, safety and no toxic side effects. The tumors can even be eliminated and regressd completely.

### Example 48

16 kunming mice for subcutaneous-inoculation of sarcoma S-180 were selected, half-male and half-female, randomly divided into 2 groups based on the tumor volume when the tumor size was about 5-10 mm after measuring and calculating the tumor volume. The first group was the control group, where each nude mouse was injected with 0.3 ml of normal saline. Each mouse in the second group was injected through caudal intravenous injection with 0.3 ml of 1% PBS solution of magnetic nanoparticle (with SAR of 510 W/gFe) - transferrin targeted nanoparticle drug with 20-50 nm particle size containing 60 mg of Fe prepared in said example 22, exerted *in vitro* with 0.3T of magnetic field on tumor location for 30 minutes, and placed in the alternating magnetic field of magnetic hyperthermia therapeutic apparatus to raise the temperature of tumor to 42-43 degree C for treatment of 1 hour twice. After treatment for 10 days, the aforesaid mice were killed, and the tumors were separated and weighed. The results showed that the tumor weight was 2.13 ± 0.46 g, 0.64 ± 0.42 g, for the first and the second group, respectively. The tumor inhibition rate in the therapeutic group was 70%, with a P value less than 0.01.

### Example 49

16 nude mice for subcutaneous-inoculation of colon cancer cell LS174T were selected, half-male and half-female, randomly divided into 2 groups based on the tumor volume when the tumor size was about 5-10 mm after measuring and calculating the tumor volume. The first group was the control group, where each nude mouse was injected with 0.5 ml of normal saline. Each nude mouse in the second group was injected through caudal intravenous injection with 0.5 ml of deionized water solution of magnetic nanoparticle coated with glucan (with SAR of 110 W/gFe) targeted nanoparticle drug with 20-30 nm particle size containing 100 mg of Fe prepared in said example 7, exerted *in vitro* with 0.6T of magnetic field on tumor location for 30 minutes, and placed in the alternating magnetic field of magnetic hyperthermia therapeutic apparatus to raise the temperature of tumor to 42-43 degree C for treatment of 2 hours once. After treatment for 10 days, the aforesaid nude mice were killed, and the tumors were separated and weighed. The results showed that the tumor weight of was 1.86 ± 0.39 g, 0.65 ± 0.41 g, for the first and the second group, respectively. The tumor inhibition rate in the therapeutic group was 65%, with a P value less than 0.01.

### Example 50

16 nude mice for subcutaneous-inoculation of colon cancer cell LS174T were selected, half-male and half-female, randomly divided into 2 groups based on the tumor volume when the tumor size was about 5-10 mm after measuring and calculating the tumor volume. The first group was the control group, where each nude mouse was injected with 0.3 ml of normal saline. Each nude mouse in the second group was injected through caudal intravenous injection with 0.3 ml of deionized water solution of magnetic nanoparticle with good long circulating stability *in vivo* modified by polyethylene glycol of about 10-20 nm particle size (with SAR of 210 W/gFe) prepared in said example 14, containing 60 mg of Fe, excerted *in vitro* with 0.8T of magnetic field on tumor position for 30 minutes, and placed in the alternating magnetic field of magnetic hyperthermia therapeutic apparatus to raise the temperature of tumor to 42-43 degree C for treatment of 1 hour once. After treatment for 10 days, the aforesaid nude mice were killed, and the tumors were separated and weighed. The results showed that the tumor weight was 2.23 ± 0.61 g, 0.61 ± 0.36 g, for the first and the second group, respectively. The tumor inhibition rate in the therapeutic group was 73%, with a P value less than 0.01.

### Example 51

16 nude mice for subcutaneous-inoculation of colon cancer cell LS174T were selected, half-male and half-female, randomly divided into 2 groups based on the toumor volume when the tumor size was about 5-10 mm after measuring and calculating the tumor volume. The first group was the control group, where each nude mouse was injected with 0.2 ml of normal saline. Each nude mouse in the second group was injected through caudal intravenous injection with 0.2 ml of normal saline solution of magnetic nanoparticle (with SAR of 7000 W/gFe) - anti-CEA antibody targeted nanoparticle drug of 50-100 nm particle size prepared in said example 18, containing 30 mg of Fe, exerted *in vivo* 0.3T magnetic field on tumor for 60 minutes, and placed in the alternating magnetic field of magnetic hyperthermia therapeutic apparatus to raise the temperature of tumor to 45-48 degree C for treatment of 1 hour once. The tumor was completely eliminated and regressed with one month after treatment. No tumor recurrence or metastasis was observed within 12 months after treatment. The weight and life-span of nude mice after treatment were basically the same as to those of nude mice without inoculated tumors in the control group. Animal tests showed that the magnetic hyperthermia of the tageted nanoparticle drug could kill and cure the maglinant tumors completely, without any notable toxic side effects.

### Example 52

16 kunming mice for subcutaneous-inoculation of sarcoma S-180 were selected, half-male and half-female, randomly divided into 2 groups based on tumor volume when the tumor size was about 5-10 mm after measuring and calculating the tumor volume. The first group was the control group, where each nude mouse was injected with 0.3 ml of normal saline. Each mouse in the second group was injected intraperitoneally with 0.3 ml of injection formulation containing 60 mg superparamagnetic iron oxide nanoparticle (with SAR of 5000 W/gFe) - transferrin targeted nanoparticle drug with 30-50 nm particle size in said example 22, and placed in the alternating magnetic field of magnetic hyperthermia therapeutic apparatus to raise the temperature of tumor to 95 degree C for treatment for 1 minute once. After treatment for 10 days, the aforesaid nude mice were killed, and the tumors were separated and weighed. The results showed that the tumor weight was 2.09 ± 0.46 g, 0.44 ± 0.31 g, for the first and the second group, respectively. The tumor inhibition rate in the therapeutic group was 79%, with a P value less than 0.01.

### Example 53

16 nude mice for subcutaneous-inoculation of liver cancer cell BEL7404 were selected, half-male and half-female, randomly divided into 2 groups based on the tumor volume when the tumor size was about 5-10 mm after measuring and calculating the tumor volume. The first group was the control group, where each nude mouse was injected with 0.3 ml of normal saline. Each nude mouse in the second group was injected intraperitoneally with 0.3 ml of injection formulaltion containing 80 mg superparamagnetic iron oxide nanoparticle (with SAR of 1500 W/gFe) - folate targeted nanoparticle drug with 30-50 nm particle size prepared in said example 25, and placed in the alternating magnetic field of magnetic hyperthermia therapeutic apparatus to raise the temperature of tumor to 42-43 degree C for treatment for 1 hour once. After treatment for 10 days, the aforesaid nude mice were killed, and the tumors were separated and weighed. The results showed that the tumor weight was 2.03 ± 0.57 g, 0.54 ± 0.36 g, for the first and the second group, respectively. The tumor inhibition rate in the therapeutic group was 73%, with a P value less than 0.01.

### Example 54

16 nude mice for subcutaneous-inoculation of liver cancer cell BEL7404 were selected, half-male and half-female, randomly divided into 2 groups based on the tumor volume when the tumor size was about 5-10 mm after measuring and calculating the tumor volume. The first group was the control group, where each nude mouse was injected with 0.2 ml of normal saline. Each nude mouse in the second group was injected intraperitoneally with 0.2 ml of injection formulation containing 50 mg superparamagnetic iron oxide nanoparticle (with SAR of 6000 W/gFe) - folate targeted nanoparticle drug with 20-40 nm particle size prepared in said example 26, and placed in the alternating magnetic field of magnetic hyperthermia therapeutic apparatus to raise the temperature of tumor to 42-43 degree C for treatment for 1 hour once. After treatment for 10 days, the aforesaid nude mice were killed, and the tumors were separated and weighed. The results showed that the tumor weight was 2.13 ± 0.65 g, 0.66 ± 0.32 g, for the first and the second group, respectively. The tumor inhibition rate in the therapeutic group was 69%, with a P value less than 0.01.

### Example 55

16 nude mice for subcutaneous-inoculation of gastric cancer cells SGC-7901 were selected, half-male and half-female, randomly divided into 2 groups based on the tumor volume when the tumor size was about 5-10 mm after measuring and calculating the tumor volume. The first group was the control group, where each nude mouse was injected with 0.2 ml of normal saline. Each nude mouse in the second group was injected intraperitoneally with 0.2 ml of injection formulation containing 30 mg magnetic nanoparticle (with SAR of 500 W/gFe) - folate targeted nanoparticle drug with 20-40 nm particle size prepared in said example 27, and placed in the alternating magnetic field of magnetic hyperthermia therapeutic apparatus to raise the temperature of tumor to 42-43 degree C for treatment for 1 hour once. After treatment for 10 days, the aforesaid nude mice were killed, and the tumors were separated and weighed. The results showed that the tumor weight was 1.76 ± 0.39 g, 0.58 ± 0.41 g, for the first and the second group, respectively. The tumor inhibition rate in the therapeutic group was 67%, with a P value less than 0.01.

### Example 56

16 nude mice for subcutaneous-inoculation of colon cancer cells LS 174T were selected, half-male and half-female, randomly divided into 2 groups based on the tumor volume when the tumor size was about 5-10 mm after measuring and calculating the tumor volume. The first group was the control group, where each nude mouse was injected with 0.2 ml of normal saline. Each nude mouse in the second group was injected through caudal intravenous injection with 0.2 ml of normal saline solution of iron oxide nanoparticle (with SAR of 7000 W/gFe) - anti-CEA antibody targeted nnaoparticle drug with 20-40 nm particle size prepared in said example 18, containing 30 mg of Fe, and placed in the alternating magnetic field of magnetic hyperthermia therapeutic apparatus to raise the temperature of tumor to 42-45 degree C for treatment for 1 hour for three times. The tumor was completely eliminated and regressed after treatment for 1 month. No tumor recurrence or metastasis was observed within 12 months after treatment. The weight and life-span of nude mice after treatment were basically the same as to those of nude mice without inoculated tumors in the control group. Animal tests showed that, the magnetic hyperthermia treatment using the tageted nanoparticle drugs of the present invention can kill and cure the maglinant tumors completely, without any notable toxic side effects.

### Example 57

24 nude mice for subcutaneous-inoculation of liver cancer cells BEL7404 were selected, half-male and half-female, randomly divided into 3 groups based on the tumor volume when the tumor size was about 10 mm after measuring and calculating the tumor volume. The first group was the control group, where each nude mouse was injected with 1.0 ml of normal saline for three times. Each nude mouse in the second group was injected through caudal intravenous injection for three times with 1.0 ml of normal saline solution of magnetic iron oxide nanoparticle (particle size of about 350-400 nm) (with SAR of 30 W/gFe) - folate targeted nanoparticle drug with 400 nm of particle size containing 100 mg of Fe prepared in said example 24. Each nude mouse in the third group was injected through caudal intravenous injection for three times with 1.0 ml normal saline solution of magnetic iron oxide nanoparticle (particle size of about 950-1000 nm) (with SAR of 10 W/gFe) - folate targeted nanoparticle drug with 1000 nm of particle size containing 200 mg of Fe prepared in said example 24. Both the second and third groups were placed into alternating magnetic field of magnertic hyperthermia therapeutic apparatus to raise the temperature of tumor to 42-43 degree C for the treatment for 1 hour twice. After 10 days of treatment the aforesaid nude mice were killed, the tumors were separated and and weighed. The results showed that the tumor weights were 2.76 ± 0.69 g, 0.24 ±0.16 g or 0.48 ± 0.33 g, for the first, the second and third group, reapectively. The tumor inhibition rates were 91% and 83% with P value of less than 0.01, for the second and third therapeutic group, respectively.

### Example 58

10 small white mice and 10 guinea pigs were selected, half-male and half-female. Their abdominal cavities were respectively injected with 0.5 ml containing 0.1 g, or 1.5 ml containing 1.0 g of magnetic nanoparticle - anti-CEA antibody targeted nanoparticle drug prepared in said example 18. No adverse reaction and weight loss were observed after 7 days and 30 days. The mice were killed, and the slices of vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatments and no heat generation was observed. Typically only 5-100 mg of magenetic nanoparticle - anti-CEA antibody targeted nanoparticle drugs were needed for tumor magnetic hyperthermia treatment. Based on the results of the aforesaid preliminary toxic side effect tests, the targeted nanoparticle drug in the present invention was biodegradabile, without toxic side effects.

### Example 59

10 small white mice and 10 guinea pigs were selected, half-male and half-female. Their abdominal cavities were respectively injected with 0.5 ml containing 0.3 g, or 1.5 ml containing 1.0 g of magnetic nanoparticle modified by decanic acid in said example 2. No adverse reaction and weight loss were observed after 7 days and 30 days. The mice were killed, and the slices of the vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatment, and no heat generation was observed. Typically only 5-100 mg of magenetic nanoparticles modified by decanic acid were needed for tumor magnetic hyperthermia treatment. Based on the results of the aforesaid preliminary toxic side effect tests, the magentic nanoparticle modifed by decanic acid in present invention was biodegradable, without toxic side effects.

### Example 60

10 small white mice and 10 guinea pigs were selected, half-male and half-female. Their abdominal cavities were respectively injected with 0.5 ml containing 0.3 g, or 1.5 ml containing 1.0 g of *in vivo* stable long circulating magnetic nanoparticle coated with liposomes and modified by PEG in said example 14. No adverse reaction and weight loss were observed after 7 days and 30 days. The mice were killed, and the slices of the vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatment and no heat generation was observed. Typically only 5-100 mg of *in vivo* stable long circulating magenetic nanoparticles coated with liposomes and modified by PEG were needed for tumor magnetic hyperthermia treatment. Based on the results of the aforesaid preliminary toxic side effect tests, the stable long circulating magentic nanoparticle coated by liposomes and modified by PEG in the present invention was biodegradable, without toxic side effects.

### Example 61

10 small white mice and 10 guinea pigs were selected, half-male and half-female. Their abdominal cavities were respectively injected with 0.5 ml containing 0.3 g, or 1.5 ml containing 1.0 g of magnetic nanoparticle - anti-human hepatoma McAb HAb18 targeted nanoparticle drug in said example 16. No adverse reaction and weight loss were observed after 7 days and 30 days. The mice were killed, and the slices of the vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatment, and no heat generation was observed. Typically only 5-100 mg of magnetic nanoparticle - anti-human hepatoma McAb HAb18 targeted nanoparticle drugs were needed for tumor magnetic hyperthermia treatment. Based on the results of the aforesaid preliminary toxic side effect tests, the targeted nanoparticle drug in the present invention was biodegradable, without toxic side effects.

### Example 62

10 small white mice and 10 guinea pigs were selected, half-male and half-female. Their abdominal cavities were respectively injected with 0.5 ml containing 0.3 g, or 1.5 ml containing 1.0 g of magnetic nanoparticle - transferrin targeted nanoparticle drug in said example 22. No adverse reaction and weight loss were observed after 7 days and 30 days. The mice were killed, and the slices of the vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatment, and no heat generation was observed. Typically only 5-100 mg of magnetic nanoparticle - transferrin targeted nanoparticle drugs were needed for tumor magnetic hyperthermia treatment. Based on the results of the aforesaid preliminary toxic side effect tests, the targeted nanoparticle drug in the present invention was biodegradable, without toxic side effects.

### Example 63

10 small white mice and 10 guinea pigs were selected, half-male and half-female. Their abdominal cavities were respectively injected with 0.5 ml containing 0.3 g, or 1.5 ml containing 1.0 g of magnetic nanoparticle - folate targeted nanoparticle drug in said example 25. No adverse reaction and weight loss were observed after 7 days and 30 days. The mice were killed, and the slices of the vital organs (such as heart, brain, spleen, kidney, intestine, liver, etc) were taken out for pathological examination. No obvious abnormality was found. The mice were killed after 30 days of observation, and the vital organs (such as heart, brain, spleen, kidney, intestine, liver, etc) were taken out for magnetic hyperthermia treatment. No heat generation was observed. Typically only 5-100 mg of magnetic nanoparticle - folate targeted nanoparticle drugs were needed for tumor magnetic hyperthermia treatment. Based on the results of the aforesaid preliminary toxic side effect tests, the targeted nanoparticle drug in the present invention was biodegradable, without toxic side effects.

## Claims

1. A targeted magnetic nanoparticle drug, comprising an effector molecule and a guidance molecule with a weight rate of effector molecule to guidance molecule of 1 : 0.0001-0.20; said effector molecule being a magnetic particle with particle size of not more than 1000 nm and specific absorption rate (SAR) of 10-7000 W/gFe; said guidance molecule comprising an antibody, a ligand or a magnetic particle with targeted magnetism function; the particle size of said targeted drug being 2-1000 nm.

2. The targeted magnetic nanoparticle drug of claim 1, wherein said the specific absorption rate (SAR) of the magnetic particle is 500-7000 W/gFe.

3. The targeted magnetic nanoparticle drug of claim 1, wherein said magnetic particle is the magnetic particle modified by a surfactant and/or a surface modifier, with a weight ratio of the magnetic particle to the surfactant and/or surface modifier of 1 : 0.0001-0.20.

4. The targeted magnetic nanoparticle drug of claim 1, wherein said surfactant and/or surface modifier comprises any one selected from sodium dodecyl sulfate, sodium oleate, decanoic acid, organic silicon coupling agent, sodium dodecyl benzene sulfonate, nucleotide or polypeptide, or a combination thereof.

5. The targeted magnetic nanoparticle drug of claim 1, wherein said magnetic particle is ferromagnetic, ferrimagnetic or superparamagnetic nanoparticle.

6. The targeted magnetic nanoparticle drug of claim 1, wherein said antibody is the antibody against a target molecule selected from a tumor-specific antigen, a tumor-associated antigen, an idiotypic determinant, a receptor of a cytokine, a hormone or a product of a tumor gene, and comprises a heterologous polyclonal antibody, a mouse monoclonal antibody or a human monoclonal antibody.

7. The targeted magnetic nanoparticle drug of claim 1, wherein said ligand includes any one selected from a folate ligand, a transferrin, a carbohydrate ligand, a polypeptide, a nucleic acid or a hormone, or a combination thereof; said folate ligand including any one selected from folic acid, folinic acid, dihydrofolic acid, tetrahydrofolic acid, tetrahydropterin, pteroylpolyglutamic acid, 2-deamino-hydroxy folate, 1-deamino-hydroxy folate, 1-denitrogen folate, 3-denitrogen folate, 8-denitrogen folate, 5-methyltetrahydro folate (5-CH₃N₄ folate), 5-formyl tetrahydro folate (5-CHOH₄ folate), antifolic acid agent methotrexate (MTX), 5,10-methylene tetrahydro folate (5,10-CHH folate, DDATHF), or a combination thereof.

8. The targeted magnetic nanoparticle drug of claim 1, wherein said antibody is any one selected from the antibody against CEA, AFP, CA19-9, CA125, PSA, Trigen, TheraCIMh-R3, Smart ID10, Vitaxin, 4B5, SS1 (dsFv) PE38, ABX-EGF, BrevaRex, CA15-3, PAP, CA50, β-hCG, SCC, and β₂M, and Rituxan, anti-VEGF, epratuzmab, anti-NSE, anti-HER2, anti-TPA antibody or anti-human hepatoma monoclonal antibody HAb18, or a combination thereof.

9. The targeted magnetic nanoparticle drug of claim 1, wherein said magnetic particle is coateded with an organic or inorganic substance on the surface.

10. The targeted magnetic nanoparticle drug of claim 9, wherein said organic substance includes liposome, polylactic acid, polycaprolactone, polyethylene glycol, glucan, chitosan, dextran or protein, and said inorganic substance is silicon dioxide, aluminium oxide, or gold.

11. The targeted magnetic nanoparticle drug of claim 10, wherein the raw material weight ratio of said magnetic particle to said organic or inorganic substance is 1 : 0.01-5.0.

12. The targeted magnetic nanoparticle drug of claim 10, wherein said drug can be made into an injection, solid or powder dosage form.

13. The method for the preparation of the targeted magnetic nanoparticle drug according to claim 1, comprising coupling a mangnetic particle and a guidance molecule in a weight ratio of 1 : 0.0001-0.20 in water or a solvent; said solvent including orgainic or inorgenaic substance, or a mixture thereof; said organic subtance being ethanol, acetic acid, toluene, or dimethylene sulfone; and said inorganic substance being phosphate.

14. The method for the preparation of the targeted magnetic nanoparticle drug according to claim 13, comprising modifying said magnetic particle with a surfactant and/or surface modifier selected from sodium dodecyl sulfate, sodium oleate, decanic acid, sodium dodecyl benzene sulfonate, organic silicon coupling agent, nucleotide, or polypeptide, with a weight ratio of the magnetic particle to the surfactant and/or surface modifier of 1 : 0.0001-0.20, followed by mixing and coupling with a guidance molecule in water or a solution.

15. The method for the preparation of the targeted magnetic nanoparticle drug according to 13, comprising coating the said magnetic particle with an organic or inorganic substance at a weight ratio of the magnetic particle to the organic or inorganic substance of 1 : 0.01-5.0, followed by mixing and coupling with a guidance molecule in a solution or water; said organic substance including liposome, polyethylene glycol, glucan, chitosan, dextran, polylactic acid, polycaprolactone, or a protein; and said inorganic substance being silicon dioxide, aluminium oxide, or gold.

16. Use of the targeted magnetic nanoparticle drug according to any one of claims 1-12 in the manufacture of a medicament for the prevention and treatment of a tumor.
